Europäisches Patentamt

**European Patent Office**

Office européen des brevets

(11) Veröffentlichungsnummer: **0 293 715**
**A2**

## EUROPÄISCHE PATENTANMELDUNG

(21) Anmeldenummer: 88108235.8

(22) Anmeldetag: 24.05.88

(51) Int. Cl.⁴: **C08G 65/28 , C08G 65/30 , C10M 111/04 , C10M 145/36 , //(C10M111/04,101:02,107:34), (C10M145/36,145:34)**

(30) Priorität: 02.06.87 DE 3718374

(43) Veröffentlichungstag der Anmeldung:
07.12.88 Patentblatt 88/49

(84) Benannte Vertragsstaaten:
**DE FR GB IT**

(71) Anmelder: **BAYER AG
Konzernverwaltung RP Patentabteilung
D-5090 Leverkusen 1 Bayerwerk(DE)**

(72) Erfinder: **Nerger, Dittmar, Dr.
Bacherhofstrasse 74
D-4150 Krefeld(DE)**
Erfinder: **Hentschel, Karl-Heinz, Dr.
Heckschenstrasse 89
D-4150 Krefeld(DE)**
Erfinder: **Rasp, Christian, Dr.
Klutstein 13
D-5060 Bergisch Gladbach(DE)**
Erfinder: **Gupta, Pramod, Dr.
Langemarckstrasse 27
D-5152 Bedburg(DE)**
Erfinder: **Kussi, Siegfried
Brandenburger Strasse 43
D-5090 Leverkusen(DE)**

(54) **Polyether, Verfahren zu ihrer Herstellung und Schmiermittel, die diese Polyether enthalten.**

(57) Hydroxylgruppen-monofunktionelle Polyether enthalten als eingebaute Endgruppen oder Monomereinheiten
a) 1 bis 30 Gew.-% eines oder mehrerer $C_4$- bis $C_{24}$-Alkylmonophenole,
b) 1 bis 30 Gew.-% eines oder mehrerer $C_8$- bis $C_{24}$-Monoalkanole,
c) 1 bis 30 Gew.-% eines oder mehrerer $C_{10}$- bis $C_{20}$-1,2-Epoxyalkane und
d) 45 bis 80 Gew.-% Propylenoxid oder eine überwiegend aus Propylenoxid bestehende Niederalkylenoxidmischung wobei die Summe der Komponenten a) bis d) 100 Gew.-% ergibt,
und weisen mittlere Molmassen von 600 bis 2500 auf.
Sie werden durch eine anionische Epoxidpolymerisation hergestellt und als Schmiermittel und Schmiermittelbestandteil verwendet.

EP 0 293 715 A2

**Polyether, Verfahren zu ihrer Herstellung und Schmiermittel, die diese Polyether enthalten.**

Aliphatische Polyether sind im allgemeinen hydrophile Körper, die sich in Wasser lösen oder bis zu einigen Gewichtsprozent Wasser aufnehmen können. Übliche Polyether sind mit hydrophoben Substanzen nicht mischbar. Das gilt insbesondere für die überwiegende Mehrzahl der Polyalkylenglykole, die als Schmierstoffe verwendet werden. So werden z.B. in Ullmanns Encyclopädie der technischen Chemie, Verlag Chemie, Weinheim, 4. Auflage, Band 20, Seite 504, Tabelle 29 Polyalkylenglykole als schlecht mit Mineralölen mischbar eingestuft.

Trotzdem wurden bereits Versuche unternommen, spezielle Polyether mit guter Mineralölmischbarkeit zu entwickeln. So beschreibt die japanische Patentveröffentlichung Nr. 50-133 205 (1975) Polyether auf der Basis von Ethylenoxid, Propylenoxid und/oder Butylenoxid und einem längerkettigen 1,2-Epoxyalkan mit bis zu 26 C-Atomen, die gegebenenfalls eine oder zwei Hydroxylendgruppen aufweisen. Aus der Veröffentlichung geht hervor, daß zur Sicherstellung der Mineralölmischbarkeit das 1,2-Epoxyalkan mit bis zu 26 C-Atomen zu etwa 40 Gew.-% oder mehr in den Polyethern enthalten sein muß.

Im Gegensatz zu Ethylenoxid und Propylenoxid sind langkettige 1,2-Epoxyalkane keine petrochemischen Primärprodukte, sondern müssen auf synthetischem Weg hergestellt werden. Der Einbau hoher Anteile an langkettigen 1,2-Epoxyalkanen in mineralölmischbare Polyether ist deshalb in technischer und wirtschaftlicher Hinsicht aufwendig und unbefriedigend.

Mineralöllösliche Polyether werden auch beschrieben in der EP-OS 0 064 236. Es handelt sich dabei um Tetrahydrofuran enthaltende Copolyether, welche nur über ein kationisches Polymerisationsverfahren zugänglich sind. Solche kationischen Polymerisationsverfahren erfordern wegen der aggressiven Katalysatoren, die dort eingesetzt werden, besondere Reaktormaterialien und apparative Ausrüstungen. Sie sind deshalb in den üblicherweise für anionische Epoxidpolymerisationen gebräuchlichen Anlagen nicht durchführbar. Daneben sind zur Erzielung guter Mineralölmischbarkeiten auch für die in der EP-OS 0 064 236 beschriebenen Polyether in der Praxis Langketten-1,2-Epoxyalkan-Anteile von über 40 Gew.-% notwendig (siehe Vergleichsbeispiele V bis VIII und Tabelle 2).

Versucht man auf der ausschließlichen Basis der aus der EP-OS 0 064 236 bekannten Polyether niedrigviskose Schmierstoffe herzustellen, z.B. solche der wichtigen Viskositätsklasse ISO-VG 68, so stellt man fest, daß hohe Verdampfungsverluste auftreten (siehe Vergleichsbeispiel IX), welche man durch übliche Mengen an Antioxidantien nur kurzfristig zurückdrängen kann. Der Zusatz größerer Antioxidansmengen ist keine Lösung des Problems, weil sich dann die Schmiermitteleigenschaften verschlechtern.

Bis jetzt gibt es also keine in technischer und wirtschaftlicher Hinsicht voll befriedigenden Polyether für das Schmierstoffgebiet.

Es wurden nun neue hydroxylgruppen-monofunktionelle Polyether gefunden, die dadurch gekennzeichnet sind, daß sie als eingebaute Endgruppen oder Monomereinheiten

a) 1 bis 30 Gew.-% eines oder mehrerer $C_4$- bis $C_{24}$-Alkylmonophenole,

b) 1 bis 30 Gew.-% eines oder mehrerer $C_8$- bis $C_{24}$-Monoalkanole,

c) 1 bis 30 Gew.-% eines oder mehrerer $C_{10}$- bis $C_{20}$-1,2-Epoxyalkane und

d) 45 bis 80 Gew.-% Propylenoxid oder eine überwiegend aus Propylenoxid bestehende Niederalkylenoxidmischung enthalten, wobei die Summe der Komponenten a) bis d) 100 Gew.-% ergibt, und daß sie mittlere Molmassen von 600 bis 2500 aufweisen.

Vorzugsweise betragen der Anteil der Komponenten a) und b) jeweils 5 bis 15 Gew.-%, der Anteil der Komponente c) 5 bis 20 Gew.-%, der Anteil der Komponente d) 50 bis 65 Gew.-% und die mittlere Molmasse 700 bis 1500. Die Molmasse ist dabei vorzugsweise die gewichtsgemittelte Molmasse, wie sie beispielsweise nach der GPC-Methode bestimmt wird.

Als Komponente a) kommen z.B. einfach oder mehrfach alkylsubstituierte Monophenole in Frage. Bei mehrfach alkylsubstituierten Monophenolen ist es ausreichend, wenn einer der Alkylsubstituenten zwischen 4 und 24 C-Atome aufweist. Der Alkylsubstituent oder, bei mehreren Alkylsubstituenten derjenige mit der längsten Kohlenstoffkette, kann sich in o-, m- oder p-Position zur phenolischen OH-Gruppe befinden. Bevorzugt sind dabei die m- und p-Positionen. Vorzugsweise sind sämtliche vorhandenen Alkylreste gesättigt. Es können jedoch auch ungesättigte Alkylreste vorliegen, insbesondere solche, die die Oxidationsbeständigkeit des Polyethers nicht zu stark negativ beeinflussen. Bevorzugte Monophenole enthalten Alkylsubstituenten mit 10 bis 24 C-Atomen. Beispiele für die Komponente a) sind monoalkylsubstituierte Monophenole wie p-Butylphenol, p-t-Butylphenol, p-Amylphenol, p-t-Amylphenol, p-Octylphenol, p-Nonylphenol (wobei der Nonylrest z.B. abgeleitet sein kann von 1-Nonen oder von technischen Propylentrimeren), p-Dodecylphenol (wobei der Dodecylrest z.B. abgeleitet sein kann von 1-Dodecen, von technischen Propylentetrameren oder von technischen Isobutylentrimeren), Hexadecylphenole, Octadecylphenole und von Cashew-Nußschalenöl abgeleitete Monophenole mit ungesättigter oder, z.B. infolge Hydrierung, gesättigter aliphatischer Seitenkette in m-Position, dialkylsubstituierte Monophenole wie t-Butylkresole, Nonylkresole, Dodecylkresole und Dinonylphenole und trialkylsubstituierte Phenole wie 4-t-Butyl-2,6-dimethylphenol. Dodecylphenole sind als Komponente a) besonders bevorzugt.

Als Komponente b) kommen z.B. geradkettige oder verzweigte, gesättigte oder, sofern die Oxidationsbeständigkeit der Polyether nicht zu stark negativ beeinflußt wird, ungesättigte aliphatische Monoalkohole in Frage, jeweils mit 8 bis 24 C-Atomen. Bevorzugte Beispiele sind n-Octanol, Isooctanol, Isononanol (z.B. in Form eines Oxoalkohols auf Diisobuten-Basis), Decanole, Dodecanole, Isotridecanol (z.B. in Form eines Oxoalkohols auf der Basis von technischen Propylentetrameren), Cetylalkohol, Stearylalkohol, $C_{12}$- bis $C_{20}$-Oxoalkohole und $C_{16}$- bis $C_{24}$-Guerbetalkohole (z.B. Relanit® von Fa. Henkel, Düsseldorf). Besonders bevorzugt sind $C_{12}$- bis $C_{18}$-Alkanole wie Isotridecanol und Oxoalkohole mit 12 bis 18 C-Atomen.

Als Komponente c) kommen beispielsweise 1,2-Epoxydecan, 1,2-Epoxydodecan, 1,2-Epoxyhexadecan, 1,2-Epoxyoctadecan, sowie Mischungen davon in Frage.

Als Komponente c) kommen auch Glycidylether geradkettiger oder verzweigter $C_4$- bis $C_{24}$-Alkanole in Frage, wie 2-Ethylhexyl-, Octyl-, Decyl-, Isotridecyl-, Hexadecyl-und Octadecyl-glycidylether. Bevorzugt sind die 1,2-Epoxyalkane.

Sofern es sich bei der Komponente d) um überwiegend aus Propylenoxid bestehende Niederalkylenoxidmischungen handelt kommen beispielsweise Alkylenoxidmischungen in Frage, die über 60 Gew.-% Propylenoxid und bis zu 40 Gew.-% $C_4$- bis $C_6$-Alkylenoxide enthalten. Vorzugsweise handelt es sich bei der Komponente d) um Propylenoxid.

Die erfindungsgemäßen Polyether stellen im allgemeinen Gemische verschiedener Polyethermonoole mit unterschiedlichen Endgruppen dar.

Man kann erfindungsgemäße Polyether beispielsweise erhalten, indem man die Alkylmonophenole (Komponente a)) und die Alkanole (Komponente b)) zusammen in dem Molverhältnis als Starter für eine anionische Epoxidpolymerisation (Epoxide = Komponenten c) und d)) einsetzt, wie es auch als Molverhältnis der Monoetherendgruppen im Polyether-Endprodukt gewünscht wird. Man kann auch so verfahren, daß man die Polyether-Endprodukte mit dem bestimmten Monoetherendgruppen-Molverhältnis aus zwei oder mehr derartigen Polyethern mit jeweils anderen Endgruppen-Molverhältnissen zusammenmischt. So ist es beispielsweise möglich, separat jeweils einen Polyether mit einer Alkylmonoether-Endgruppe und einen solchen mit einer Alkylphenolmonoether-Endgruppe im angestrebten Endgruppen-Molverhältnis nachträglich zu vermischen. Die erstgenannte Herstellungsmöglichkeit ist bevorzugt.

Im Prinzip ist es auch möglich, den Einbau der Alkylphenyloxy-Gruppen (Komponente a)) über Alkylphenylglycidylether als Comonomere zu bewerkstelligen, beispielsweise durch eine anionische Copolymerisation von Propylenoxid, langkettigem 1,2-Epoxyalkan (und/oder Alkyl-glycidylether) und Alkylphenyl-glycidylether enthaltenden Monomermischungen in Gegenwart eines $C_4$-bis $C_{24}$-Alkanols.

Der Einbau der langkettigen 1,2-Alkylenoxid-Monomereinheiten (Komponente c)) im Verhältnis zum Propylenoxid kann sowohl statistisch, als auch in Blöcken, als auch einem Verteilungsgradienten folgend ("tapered copolymers") realisiert werden. Es kann in manchen Fällen vorteilhaft sein, die Alkylenoxideinheiten der Komponente c) als Block an das Hydroxylende der Polyethermonoole anzubauen. In der Regel ist ein statistischer Einbau bevorzugt.

Die Durchführung einer anionischen Epoxidpolymerisation ist dem Fachmann im Prinzip bekannt (siehe z.B. Houben-Weyl, Band 14/2, Seite 425 ff (1963); Kirk-Othmer, Band 18, Seite 624 und 638 bis 641 (1982) und Ullmann, Encyclopädie der technischen Chemie, Band 19, Seiten 33 bis 34 und 36 (1981)). Bei der Herstellung der erfindungsgemäßen Polyether ist darauf zu achten, daß flüchtige Beimengungen und Verunreinigungen besonders sorgfältig entfernt werden, beispielsweise durch Strippen, Nachbehandlung in einem Dünnschichtverdampfer oder Ausdestillieren im Hochvakuum bis zu hohen Temperaturen, z.B. maximal bis 220°C. Andernfalls können beim Gebrauch als Schmiermittel zusätzliche Verdampfungsverluste auftreten, die nicht auf Abbau- oder Zersetzungseffekten beruhen.

Die erfindungsgemäßen Polyether zeichnen sich dadurch aus, daß sie auch bei niedriger Viskosität mittel- bis langfristig, beispielsweise bei Temperaturen im Bereich 150 bis 200°C über Zeiträume von mindestens 6 Monaten, niedrige Verdampfungsverluste und hohe thermische und thermooxidative Beständigkeit aufweisen. Sie haben außerdem den Vorteil, nach einem technisch einfachen anionischen Polymerisationsverfahren zugänglich zu sein und geringere Mengen langkettiger Epoxide zu benötigen als bisher. Überraschend ist dabei, daß diese Effekte mit phenolischen Struktureinheiten aufweisenden Polyethern erzielt werden, obwohl allgemein bekannt ist, daß rein aliphatische Strukturen besonders günstige Schmiermitteleigenschaften verleihen (siehe z.B. Gunderson und Hart, Synthetic Lubricants, Reinhold Publ. Corp. New York, Seite 32 (1962) und Chemtech, Dezember 1986, Seiten 752 bis 755).

Die erfindungsgemäßen Polyether lassen sich, gegebenenfalls nach dem Zusatz üblicher Additive, als Schmiermittel oder Schmiermittelkomponente verwenden. Die vorliegende Erfindung betrifft deshalb auch Schmiermittel, welche die erfindungsgemäßen Polyether enthalten. Mit anderen Polyethern vermischt verbessern erfindungsgemäße Polyether deren thermooxidative Stabilität. Durch Zugabe von Antioxidantien wird diese Stabilität auf synergistische Weise verstärkt, wie in den Beispielen detailliert dargelegt ist. Schließlich lassen sich aufgrund der besonderen Lösungseigenschaften der erfindungsgemäßen Polyether zusammen mit Mineralölen und/oder Poly-α-olefinen teil- oder vollsynthetische Schmiermittel mit hohem Leistungsprofil formulieren. Solche Mischungen enthalten bevorzugt

50 bis 95 Gew.-Teile einer Mineralölfraktion mit Schmierviskosität oder eines Poly-α-olefins,

5 bis 50 Gew.-Teile der erfindungsgemäßen Polyether und

0 bis 10 Gew.-Teile übliche Schmierstoffadditive.

Unter dem Begriff "Schmierviskosität" wird eine Stoffeigenschaft verstanden, die Stoffe mit für Schmiermittel nicht ausreichender Viskosität ausschließt. Im allgemeinen wird eine Mindestviskosität (gemessen im Schmierspalt bei Belastung) von mindestens 2 $mm^2/s$ gefordert.

Besonders bevorzugt enthalten solche Mischungen 15 bis 35 Gew.-Teile der erfindungsgemäßen Polyether. Weiterhin kann man die erfindungsgemäßen Polyether auch vorteilhaft mit Schmierstoffen auf der Basis von Estern, Phosphaten, Glykolen und Polyglykolen vermischen, beispielsweise 5 bis 50 Gew.-% der erfindungsgemäßen Polyether mit 50 bis 95 Gew.-% Schmierstoffen auf anderer Basis und gegebenenfalls üblichen Mengen üblicher Additive.

Erfindungsgemäße Polyether enthaltende Schmierstoffe und Schmierstoffmischungen können zusätzlich übliche Additive enthalten, die die Grundeigenschaften von Schmierstoffen verbessern, beispielsweise Antioxidantien, Metallpassivatoren, Rostinhibitoren, Viskositäts index-Verbesserer, Stockpunkterniedriger, Dispergiermittel, Detergentien, Hochdruckzusätze und/oder Verschleiß-Additive.

Die Antioxidantien können z.B. Phenolderivate sein, insbesondere alkylierte Monophenole, alkylierte Hydrochinone, hydroxylierte Thiodiphenylether, Alkylidenbisphenole, Benzylphenolverbindungen, Acylaminophenole, Ester oder Amide der β-(3,5-Di-tert.-butyl-4-hydroxyphenyl)-propionsäure oder Ester der β-(5-tert.-butyl-4-hydroxy-3-methylphenyl)-propionsäure. Alle diese Phenolderivate können Alkylgruppen enthalten. Es kann sich dabei beispielsweise um Methyl-, Ethyl-, n-Butyl-, i-Butyl-, t-Butyl-, Octyl-, Nonyl-, Dodecyl-, Octadecyl-, Cyclopentyl-, Cyclohexyl- und Methylcyclohexylgruppen handeln. Gegebenenfalls können noch weitere Substituenten enthalten sein, beispielsweise Methoxygruppen. Bei den Estern kann es sich beispielsweise um solche mit $C_1$-bis $C_{20}$-Mono- oder Polyalkoholen handeln, insbesondere um Ester von Methanol, Neopentylglykol und Pentaerythrit. Bei den Amiden kann es sich beispielsweise um solche auf der Basis von Trimethylendiamin, Hexamethylendiamin oder Hydrazin handeln.

Charakteristische Vertreter der genannten Klassen von Phenolderivaten sind beispielsweise 2,6-Di-tert.-butyl-4-methylphenol, 2,6-Di-tert.-butyl-4-methoxyphenol, 2,2'-Thio-bis-(6-tert.-butyl-4-methylphenol), 2,2'-Methylen-bis-(6-tert.-butyl-4-methylphenol), 2,2'-Ethyliden-bis-(6-tert.-butyl-4-isobutylphenol), 1,3,5-Tri-(3,5-di-tert.-butyl-4-hydroxybenzyl)-2,4,6- trimethylbenzol, Bis-(4-tert.-butyl-3-hydroxy-2,6-dimethylbenzyl)-dithiolterephthalat, 3,5-Di-tert.-butyl-4-hydroxybenzyl-phosphonsäure-dioctadecylester und 4-Hydroxy-laurinsäureanilid.

·Bei den Antioxidantien kann es sich auch um Amine handeln, beispielsweise um N,N'-Di-isopropyl-p-phenylendiamin, N-Phenyl-1-naphthylamin, 4-Butyrylamino-phenol, 2,4'-Diamino-diphenylmethan oder substituierte Diphenylamine.

Bei den Metallpassivatoren kann es sich beispielsweise um Benztriazol. Tetrahydrobenztriazol, 2-Mercaptobenzthiazol, Salicyliden-propylendiamin und Salze von Salicylaminoguanidinen handeln. Als Rost-Inhibitoren sind beispielsweise organische Säuren, deren Ester, deren Metallsalze und deren Anhydride, stickstoff-, phosphor- und schwefelhaltige Verbindungen geeignet, wie N-Oleoyl-sarcosin, Blei-naphthenat, Dodecenylbernsteinsäure-anhydrid, 4-Nonylphenoxy-essigsäure, öllösliche Alkylammoniumcarboxylate, substituierte Imidazoline und Oxazoline, Aminsalze von Phosphorsäurepartialestern und Bariumdinonylnaphthalin-sulfonate.

Als Viskositätsindexverbesserer kommen beispielsweise Polymethacrylate, Vinylpyrrolidon-methacrylat-Copolymere, Polybutene, Olefin-Copolymere und Styrol-acrylat-Copolymere in Frage, sowie Ester aromatischer Dicarbonsäuren mit Poly-tetrahydrofuran-Diolen (siehe DE-OS 32 21 137).

Geeignete Stockpunktserniedriger sind beispielsweise Polymethacrylate und alkylierte Naphthalinderivate.

Beispiele für Dispergiermittel und/oder Tenside sind Polybutenylbernsteinsäure-imide, Polybutenylphosphonsäurederivate und basische Magnesium-, Calcium- und Bariumsulfonate und -phenolate.

Bei den Hochdruck- und/oder verschleißmindernden Additiven kann es sich beispielsweise um Schwefel, Phosphor oder Halogene enthaltende Verbindungen handeln, wie geschwefelte pflanzliche Öle, Zinkdialkyldithiophosphonate, Tritolylphosphat, chlorierte Paraffine und Alkyl- und Aryldisulfide.

Insgesamt sind in den erfindungsgemäße Polyether enthaltenden Schmiermitteln Additive im allgemeinen nicht zu mehr als 10 Gew.-% enthalten und einzelne Additivkomponenten zu nicht mehr als 3 Gew.-%. Die folgenden Beispiele erläutern die vorliegende Erfindung, ohne sie darauf zu begrenzen.

Beispiele

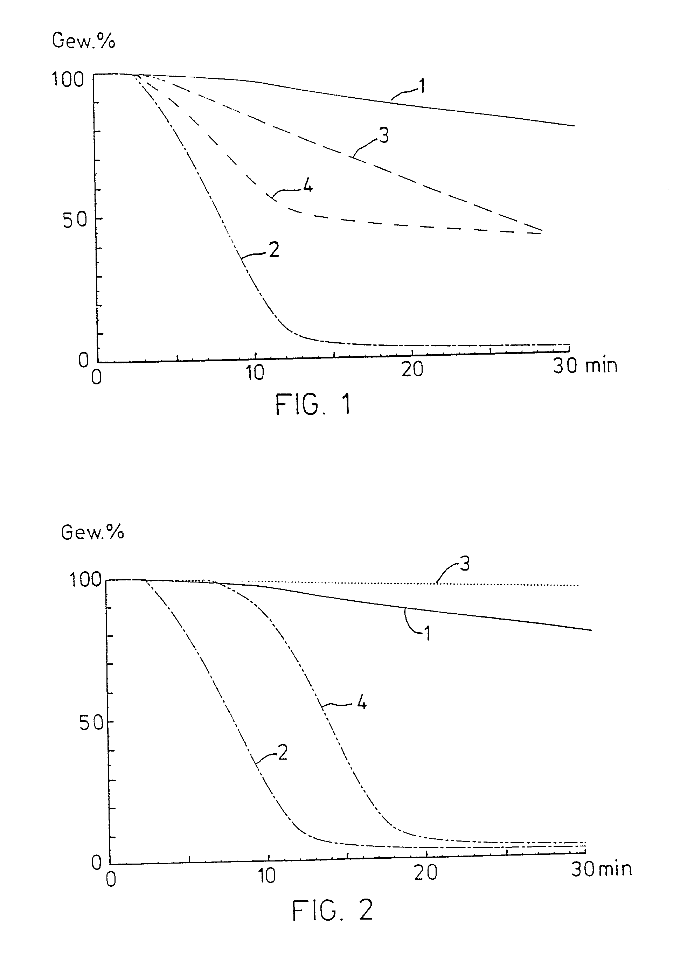

Die Eigenschaften der nach den folgenden Beispielen und Vergleichsbeispielen erhaltenen Produkte sind insbesondere aus den Figuren 1 bis 4 und den Tabellen 1 und 2 mit zugehörenden Erläuterungen ersichtlich.

Beispiel 1

Eine Mischung aus 109,2 g Dodecylphenol (0,417 Mol), 116,7 g Isotridecanol (0,584 Mol) und 56 g einer 10 %igen wäßrigen Kalilauge (0,1 Mol) wurde mit Toluol azeotrop entwässert und danach destillativ vom Toluol befreit. Mit der so erhaltenen Mischung wurde ein 2 l-Autoklav beschickt, der unter Rühren auf 170°C aufgeheizt wurde. Bei dieser Temperatur wurden innerhalb von 3 Stunden zunächst 533,6 g Propylenoxid (9,2 Mol), danach innerhalb von 30 Minuten 147,2 g 1,2-Epoxydodecan (0,8 Mol) eindosiert. Nach einer 2-stündigen Nachreaktionszeit bei 170 bis 180°C wurde abgekühlt, der Autoklaveninhalt entleert und durch Einleiten von Kohlendioxidgas neutralisiert. Abschließend wurde das Gemisch bei 200°C und 1 h Pa ausdestilliert und über ein Anschlämmfilter abgedrückt. Die Ausbeute betrug 834,0 g (ca. 92 % der Theorie), die OH-Zahl des Produktes 75.

Beispiel 2

Eine Mischung aus 104,8 g Dodecylphenol (0,4 Mol), 80,0 g Isotridecanol (0,4 Mol) und 45 g einer 10 %igen wäßrigen Kalilauge (0,08 Mol) wurde mittels Toluol azeotrop entwässert und danach destillativ wieder vom Toluol befreit. Mit dieser Mischung wurde ein 2 l-Autoklav beschickt und weiterhin so verfahren, wie in Beispiel 1 beschrieben, wobei 519,7 g Propylenoxid (8,96 Mol) und 117,8 g 1,2-Epoxydodecan (0,64 Mol) nacheinander eingeleitet wurden. Die Ausbeute betrug 746,6 g (ca. 90,8 % der Theorie), die OH-Zahl des Produktes 66.

Beispiel 3

Eine Mischung aus 65,5 g Dodecylphenol (0,25 Mol), 150,0 g Isotridecanol (0,75 Mol) und 56 g einer 10 %igen wäßrigen Kalilauge (0,1 Mol) wurde mittels Toluol azeotrop entwässert und anschließend destillativ wieder vom Toluol befreit. Mit dieser Mischung wurde ein 2 l-Autoklav beschickt und weiter so verfahren, wie in Beispiel 1 beschrieben. Es wurden 533,6 g Propylenoxid (9,2 Mol) und danach 147,2 g 1,2-Epoxydodecan (0,8 Mol), letzteres während 50 Minuten, zugegeben. Die Ausbeute betrug 835,3 g (ca. 93,2 % der Theorie), die OH-Zahl des Produktes 74.

Beispiel 4

Eine Mischung aus 102,2 g Dodecylphenol (0,417 Mol), 116,7 g Isotridecanol (0,584 Mol) und 56 g einer 10 %igen wäßrigen Kalilauge (0,1 Mol) wurde mittels Toluol azeotrop entwässert und anschließend destillativ wieder vom Toluol befreit. Mit dieser Mischung wurde ein 2 l-Autoklav beschickt und, wie in Beispiel 1 beschrieben, weitergearbeitet. Im Verlaufe von 3 Stunden wurde ein Gemisch aus 556,8 g Propylenoxid (9,6 Mol) und 73,6 g 1,2-Epoxydodecan (0,4 Mol) zudosiert. Die Ausbeute betrug 792 g (ca. 92,5 % der Theorie), die OH-Zahl des Produktes 75.

Beispiel 5

Das Beispiel 4 wurde wiederholt, jedoch wurden 163,8 g Dodecylphenol (0,625 Mol), 175,1 g Isotridecanol (0,876 Mol), 84·g einer 10 %igen wäßrigen Kalilauge (0,15 Mol), 800,4 g Propylenoxid (14,3 Mol) und 220,8 g 1,2-Epoxydodecan (1,2 Mol) eingesetzt. Die Ausbeute betrug 1279,9 g (ca. 94,1 % der Theorie), die OH-Zahl des Produktes 67.

Beispiel 6

In 704 g des Polyethers, der entsprechend Vergleichsbeispiel I erhalten worden war, wurden 0,9 g Natrium aufgelöst. Nach dem Aufheizen dieser Mischung auf 150° C wurden unter Stickstoff innerhalb von 30 Minuten 88,3 g 1,2-Epoxydodecan zugetropft und 3 Stunden bei 150° C nachgerührt. Nach dem Abkühlen wurde mit 19,1 g einer 10 %igen Schwefelsäure neutralisiert und danach der Ansatz bei 200° C und 1 h Pa ausdestilliert und über ein Anschlämmfilter abgedrückt. Die OH-Zahl des so erhaltenen Produktes betrug 59, die Ausbeute 739,2 g (ca. 93,3 % de Theorie).

Beispiel 7

Es wurde verfahren wie in Beispiel 6, jedoch wurden nur 44,2 g 1,2-Epoxydodecan eingesetzt. Die OH-Zahl des Produktes betrug dann 61, die Ausbeute 708,4 g (ca. 94,7 % der Theorie).

Beispiel 8

2 g Natrium (0,087 Mol) wurden in 170 g Isotridecanol (0,85 Mol) aufgelöst, diese Mischung in einen 2 l-Autoklaven gegeben und unter Rühren auf 170° C aufgeheizt. Innerhalb von 3 Stunden wurden dann 552,2 g Propylenoxid (9,52 Mol), danach innerhalb von 40 Minuten 187,7 g p-Nonylphenol-glycidylether (0,68 Mol) zudosiert und noch 2 Stunden bei 180° C nachreagieren gelassen. Nach dem Abkühlen wurde der Inhalt des Autoklaven entleert. Die Reaktionsmischung wurde mit einer dem eingesetzten Natrium äquivalenten Menge 10 %iger Schwefelsäure neutralisiert, bei 200° C und 1 h Pa ausdestilliert und danach über ein Anschlämmfilter abgedrückt. Die Ausbeute betrug 812,5 g (ca. 89,3 % der Theorie), die OH-Zahl des Produktes 79.

Beispiel 9

In einer Versuchsapparatur wurden 722,7 g Dodecylphenol, 772 g Isotridecanol und 66,7 g 45 %ige Kalilauge vermischt. Das Wasser wurde von dieser Mischung abdestilliert, zuletzt bei einem Vakuum von 1 h Pa und 110° C. Innerhalb von 10 Stunden wurden dann bei 100 bis 110° C 3531,1 g Propylenoxid, danach innerhalb von 1,5 Stunden 974,2 g 1,2-Epoxydodecan zugeführt. Anschließend wurde durch Zugabe von 2073 g einer 12,5 %igen Schwefelsäure neutralisiert. Danach wurde das Wasser abdestilliert und das Gemisch zuletzt 3 Stunden lang bei 105° C und 1 h Pa ausgeheizt und abschließend unter Stickstoff abkühlen gelassen. Die OH-Zahl des so erhaltenen Produktes war 66, die Ausbeute 5860 g (ca. 97,7 % der Theorie).

Beispiel 10

Das gemäß Beispiel 5 erhaltene Produkt wurde zu gleichen Gew.-Teilen mit dem gemäß Vergleichsbeispiel IV erhaltenen Produkt gemischt. Von diesem Gemisch wurde gemäß DIN 51 581 (Noack-Test) der Verdampfungsverlust zu 5,8 % ermittelt. Dieser Massenverlust liegt deutlich unter dem theoretisch erwarteten Wert (arithmetisches Mittel) von 12,45 %.

6

Vergleichsbeispiel I

Es wurde verfahren wie in Beispiel 9, jedoch wurden 4505,3 g Propylenoxid während 14,5 Stunden zugegeben und kein 1,2-Epoxydodecan. Das erhaltene Produkt wies eine OH-Zahl von 64 auf. Die Ausbeute betrug 5790 g (ca. 96,5 % der Theorie).

Vergleichsbeispiel II

Eine Mischung aus 109,3 g Dodecylphenol (0,417 Mol), 116,6 g Isotridecanol (0,583 Mol) und 56 g einer 10 %igen wäßrigen Kalilauge (0,1 Mol) wurde mittels Toluol azeotrop entwässert und danach destillativ vom Toluol befreit. Mit dieser Mischung wurde ein 2 l-Autoklav beschickt, der unter Rühren auf 170° C aufgeheizt wurde. Bei dieser Temperatur wurden innerhalb von 3 Stunden 696 g Propylenoxid (12 Mol) zugeführt. Nach einer 2-stündigen Nachreaktion bei 170 bis 180° C wurde abgekühlt, der Autoklav entleert und das Reaktionsgemisch durch eine äquivalente Menge 10 %iger wäßriger Schwefelsäure neutralisiert. Danach wurde bis zu 200° C bei 1 h Pa andestilliert und schließlich über ein Anschlämmfilter abgedrückt. Es wurden 880,4 g Produkt erhalten (ca. 95,5 % der Theorie), das eine OH-Zahl von 76 aufwies.

Vergleichsbeispiel III

Es wurde verfahren wie im Vergleichsbeispiel II, jedoch wurden 27,4 g Dodecylphenol (0,105 Mol), 93,3 g Isotridecanol (0,467 Mol), 6,7 g festes Kaliumhydroxid-Pulver (0,12 Mol) und 649,6 g Propylenoxid (11,2 Mol) umgesetzt und aufgearbeitet. Es wurden 729,5 g Produkt erhalten (ca. 94,7 % der Theorie). Die OH-Zahl des Produktes betrug 83.

Vergleichsbeispiel IV

Eine Mischung aus 1015 g "Alfol® 12/18" (das ist ein Fettalkoholgemisch im Bereich $C_{12}$ bis $C_{18}$, wie es von der Condea-Chemie GmbH, Brunsbüttel, bezogen werden kann, mit einer mittleren Molmasse von 211; d.h. 1015 g entsprechen ca. 4,8 Mol) und 53,3 g einer 45 %igen wäßrigen Kalilauge (0,428 Mol) wurde mittels Toluol azeotrop entwässert und anschließend wieder vom Toluol befreit. Mit dieser Mischung wurde ein Reaktor beschickt, der unter Rühren auf 100 bis 110° C erwärmt wurde. Bei dieser Tempeatur wurden innerhalb von 23 Stunden 4985 g Propylenoxid (85,95 Mol) zugefügt. Nach dem Abkühlen wurde mit einer äquivalenten Menge 10 %iger wäßriger Schwefelsäure neutralisiert und dieser bei 40° C und 1 hPa ausdestilliert.

Dann wurde das Produkt über ein Anschlämmfilter abgedrückt. Die Ausbeute betrug 5600 g (ca. 93 % der Theorie), die OH-Zahl 47.

Erläuterung der Abbildungen:

Die Fig. 1 und 2 zeigen die Ergebnisse von Isothermogravimetriemessungen, die mit einer Thermowaage des Typs TGS-2 (Hersteller: Bodenseewerk Perkin-Elmer und Co. GmbH, Überlingen/Bodensee) bei 200° C unter Luft durchgeführt wurden.

Fig. 1:

Die Kurve 1 wurde mit einem erfindungsgemäßen Polyether ermittelt, der gemäß Beispiel 5 hergestellt worden war. Die Kurve 2 wurde mit einem Polyether ermittelt, der gemäß Vergleichsbeispiel IV hergestellt worden war. Die Kurve 3 wurde mit einem gewichtsmäßigen 1:1-Gemisch der Polyether ermittelt, die gemäß dem Beispiel 5 und dem Vergleichsbeispiel IV hergestellt worden waren. Die Kurve 4 stellt das rechnerisch ermittelte arithmetische Mittel der Kurven 1 + 2 dar.

Man ersieht aus Fig. 1, daß der erfindungsgemäße Polyether wesentlich langsamer Gewichtsverluste erleidet als der Vergleichs-Polyether (Kurven 1 und 2). Außerdem sieht man, daß ein gewichtsmäßiges 1:1-Gemisch dieser Polyether einen langsameren Gewichtsverlust erleidet als dem arithmetischen Mittel der Gewichtsverluste beider Komponenten entspricht (Kurven 3 und 4).

Beispielsweise werden jeweils 10 % Gewichtsverlust beim erfindungsgemäßen Polyether nach 16,5 Minuten, beim Vergleichspolyether nach 3,8 Minuten und bei der gewichtsmäßigen 1:1-Mischung beider nach 7,6 Minuten beobachtet, während sich für die gewichtsmäßige 1:1-Mischung aus dem arithmetischen Mittel der für die beiden Komponenten gemessenen Zeiten für 10 % Gewichtsverlust eine Zeitspanne von 5 Minuten errechnet.

Fig. 2:

Die Kurven 1 und 2 entsprechen den Kurven 1 und 2 aus Fig. 1. Die Kurve 3 wurde an einem Gemisch aus 99,5 Gew.-% des erfindungsgemäßen Polyethers hergestellt nach Beispiel 5 und 0,5 Gew.-% einer handelsüblichen Antioxidansmischung (2 Teile aralkyliertes Diphenylamin und 1 Teil 2,6-Di-tert.-butyl-p-kresol) aufgenommen, die Kurve 4 an einem Gemisch aus 99,5 Gew.-% des nach Vergleichsbeispiel IV hergestellten Polyethers und 0,5 Gew.-% der vorstehend angegebenen handelsüblichen Antioxidansmischung.

Man ersieht aus Fig. 2, daß ein üblicher Polyether (Kurve 2) durch Zusatz einer üblichen Antioxidansmischung (Kurve 4) graduell langsamer Gewichtsverluste erleidet, jedoch immer noch deutlich schneller als der erfindungsgemäße Polyether ohne Antioxidanszusatz (Kurve 1) oder gar der erfindungsgemäße Polyether mit dem Zusatz einer üblichen Antioxidansmischung (Kurve 3). Der erfindungsgemäße Polyether, mit einer üblichen Antioxidansmischung versetzt, zeigt einen außerordentlich langsamen Gewichtsverlust.

Beispielsweise werden jeweils 5 % Gewichtsverlust beim erfindungsgemäßen Polyether mit antioxidanszusatz nach 30 Minuten, beim erfindungsgemäßen Polyether ohne Antioxidanszusatz nach 11,4 Minuten, beim Vergleichspolyether mit antioxidanszusatz nach 8,2 Minuten und beim Vergleichspolyether ohne Antioxidanszusatz nach 3,2 Minuten beobachtet.

Fig. 3 und 4:

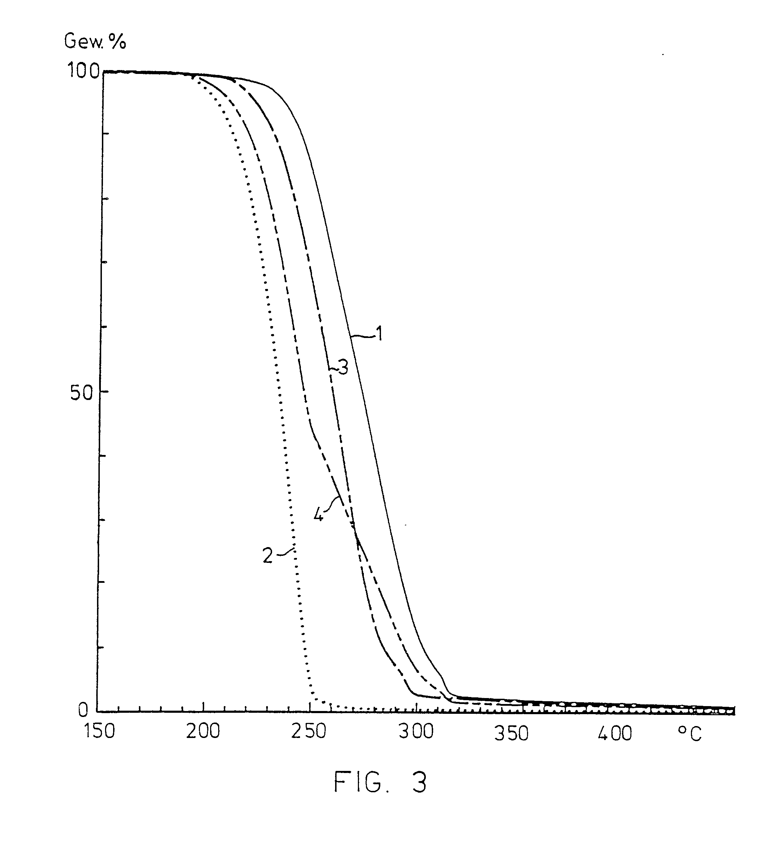

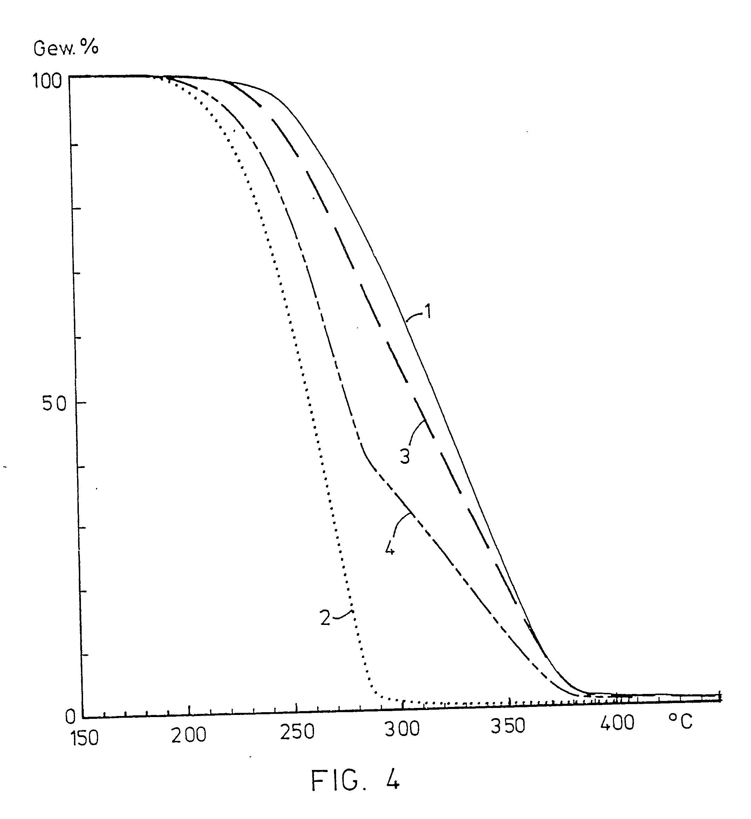

Die Fig. 3 und 4 zeigen die Ergebnisse von dynamischen Thermogravimetriemessungen, die mit der gleichen Thermowaage (wie bei Fig. 1 und 2 beschrieben) durchgeführt wurden. Die Temperatursteigerung betrug jeweils 20 °C pro Minute. Bei Fig. 3 wurde unter Luft, bei Fig. 4 under Stickstoff gemessen.

In den Fig. 3 und 4 wurde die Kurve 1 jeweils mit einem erfindungsgemäßen Polyether ermittelt, der gemäß Beispiel 5 hergestellt worden war. Die Kurve 2 wurde jeweils mit einem Polyether ermittelt, der gemäß Vergleichsbeispiel IV hergestellt worden war. Die Kurve 3 wurde jeweils mit einem gewichtsmäßigen 1:1-Gemisch der Polyether ermittelt, die gemäß dem Beispiel 5 und dem Vergleichsbeispiel IV hergestellt worden waren. Die Kurve 4 stellt jeweils das rechnerisch ermittelte arithmetische Mittel der Kurven 1 + 2 dar. Man ersieht aus den Fig. 3 und 4, daß der erfindungsgemäße Polyether (Kurve 1) erst bei höheren Temperaturen thermisch abgebaut wird als der Vergleichs-Polyether (Kurve 2). Außerdem sieht man, daß ein gewichtsmäßiges 1:1-Gemisch dieser Polyether (Kurve 3) bei höheren Temperaturen abgebaut wird als dem arithmetischen Mittel beider Komponenten entspricht (Kurve 4).

Beispielsweise werden unter Luft (Fig. 3) jeweils 5 % Gewichtsverlust beim erfindungsgemäßen Polyether bei 230 °C, beim Vergleichs-Polyether bei 202 °C und bei der gewichtsmäßigen 1:1-Mischung beider bei 220 °C beobachtet, während sich für die gewichtsmäßige 1:1-Mischung aus dem arithmetischen Mittel der für beide Komponenten gemessenen Temperaturen für 5 % Gewichtsverlust eine Temperatur von 215 °C errechnet. Die Relation dieser vier verschiedenen Temperaturen ist ganz ähnlich, wenn man jeweils unter Stickstoff mißt (Fig. 4). Die entsprechenden Zahlen sind dann 240 °C, 207 °C, 232 °C und 213 °C.

8

## Tabelle 1

| | Beispiel 1 | Vergleichs-beispiel I | Beispiel 5 | Vergleichs-beispiel IV |
|---|---|---|---|---|
| Viskosität bei 40°C [mm²/s] | 65,8 | 66,7 | 68,5 | 58,6 |
| Viskosität bei 100°C [mm²/s] | 9,7 | 10,1 | 10,3 | 11,2 |
| Pour Point (°C) | -37 | -35 | -40 | -37 |
| Viskositätsindex VIE | 129 | 136 | 136 | 192 |
| Verdampfungsverlust nach DIN 51 581 (Gew.-%) | 4,8 | 12,3 | 3,5 | 21,4 |
| Almen-Wieland-Test (Werte an der Belastungsgrenze) | | | | |
| Schweißkraft (N) | 7000 | 7000 | 10000 | 7000 |
| Reibungskraft (N) | 920 | 1600 | 1100 | 1160 |
| Temperatur (°C) | 34 | 49 | 38 | 42 |
| Reichert-Reibverschleiß-Test (bei 15 N Belastung) | | | | |
| Verschleißmarke (mm²) | 12,9 | 12,3 | 11,7 | 12,5 |
| spez. Belastbarkeit (MPa) | 23,2 | 24,3 | 25,6 | 24 |
| Löslichkeit in Mineralölen (Zusatz 50 Vol.-%) | | | | |
| zu Paraffin-basischem Grundöltyp klar bis (°C) | -14 | -13 | -16 | -10 |
| zu Gemisch-basischem Grundöltyp klar bis (°C) | -10 | -10 | -13 | + 1 |
| zu Naphthen-basischem Grundöltyp klar bis (°C) | ‹-60 | -49 | ‹-60 | -35 |
| zu Poly-α-olefin Grundöltyp klar bis (°C) | ‹-60 | -4 | ‹-60 | -10 |

EP 0 293 715 A2

EP 0 293 715 A2

Erläuterungen zu Tabelle 1

Bei den Beispielen 2 bis 4 und 6 bis 9 und den Vergleichsbeispielen II und III wurden folgende Verdampfungsverluste gemessen:

Beispiel 2      2,9
Beispiel 3      2,9
Beispiel 4      5,2
Beispiel 6      7,0
Beispiel 7      8,8
Beispiel 8      9,5
Beispiel 9      4,6
Vergleichsbeispiel II      12,2
Vergleichsbeispiel III      12,3

Bei den sonstigen Meßwerten ergeben sich bei den Produkten der Beispiele 2 bis 4 und 6 bis 9 und der Vergleichsbeispiele II und III keine nennenswerten Abweichungen gegenüber den entsprechenden Meßwerten der Produkte des Beispiels 1 und des Vergleichsbeispiels I (Ausnahme Beispiele 6 bis 8, Löslichkeit in Poly-α-olefinen).

Es ist ersichtlich, daß der stark verbesserte Verdampfungsverlust der erfindungsgemäßen Polyether keine negativen Einflüsse auf die Schmiereigenschaften und auf die Löslichkeit in Mineralölen hat (Ausnahme siehe oben).

Die reinen Mineralöle hatten die in Tabelle 2 angegebenen Trübungspunkte.

Vergleichsbeispiele V bis VIII:

Es wurde die Mineralölmischbarkeit von Polyethern entsprechend der EP-OS 0 064 236 bestimmt, die wie folgt hergestellt worden waren:

Zu einen Gemisch aus Tetrahydrofuran (THF), Alkohol und Bortrifluorid-tetrahydrofuranat (BF$_3$-T) wurde bei 55°C im Verlauf von 2 bis 6 Stunden Propylenoxid (PO), Dodecenoxid (DO) und Alkohol zugetropft. Nach 1- bis 2-stündiger Nachreaktionszeit wurde mit Soda (S) neutralisiert, das Gemisch im Vakuum bis zu 200°C bei 1 hPa ausdestilliert und über ein Anschlämmfilter abgedrückt.

Vergleichsbeispiel V

Einsatz:

234,4 g THF,
0,45 g Methanol,
30 g BF$_3$-T;
1492,9 g PO,
685,5 g DO,
8 g Methanol;
38 g S.
Produkt: OH-Zahl: 110, Dodecenoxidgehalt: 28,1 %.

Vergleichsbeispiel VI

Einsatz:

208,8 g THF,
0,4 g Methanol,
26,7 g BF$_3$-T;
1262,1 g PO,
832,4 g DO,
7,1 g Methanol;
33 g S.
Produkt: OH-Zahl: 95, Dodecenoxidgehalt: 35,6 %.


Vergleichsbeispiel VII


Einsatz:

247,5 g THF,
2,3 g Butandiol-1,4,
8,5 g BF$_3$-T;
523,0 g PO,
758,4 g Dodecenoxid,
44,5 g Butandiol-1,4 zusammen mit 302,1 g THF;
12,3 g S.
Produkt: OH-Zahl: 51, Dodecenoxidgehalt: 40,1 %.


Vergleichsbeispiel VIII


Einsatz:

382,5 g THF;
31,7 g Methanol,
13,8 g BF$_3$-T;
79,8 g PO,
712 g DO zusammen mit 409,5 g THF;
17,7 g S.
Produkt: OH-Zahl: 45, Dodecenoxidgehalt: 43,4 %.

Die so hergestellten Polyether wurden jeweils im Volumenverhältnis 1:1 mit verschiedenen Mineralölen gemischt. Von den reinen Mineralölen und den Mineralöl-Polyethergemischen wurde der Trübungspunkt nach DIN 51 597 bestimmt. Die Ergebnisse sind in Tabelle 2 zusammengefaßt.

11

## Tabelle 2

| Mineralölsorte | ohne Zusatz | Trübungspunkte [$^0$C] | | | |
|---|---|---|---|---|---|
| | | mit Zusatz von Polyether aus Vergleichsbeispiel | | | |
| | | V | VI | VII | VIII |
| Weißöl 68 | -18 | ⟩ +25 | +15 | +10 | -15 |
| Verdichteröl 100 | -8 | ⟩ +25 | ⟩ +25 | ±0 | -19 |
| Kältemaschinen- | | | | | |
| öl C/6S | ⟨ -60 | +15 | -22 | -45 | ⟨ -60 |
| Hitec E 168 | ⟨ -60 | +10 | -25 | -35 | ⟨ -60 |

## Erläuterung zu Tabelle 2

Niedrige Trübungspunkte bedeuten gute Mineralölmischbarkeit. Man erkennt, daß erst bei einem Zusatz von einem Polyether, der über 40 Gew.-% Dodecenoxid enthält (Vergleichsbeispiel VIII) etwa gleiche oder niedrigere Trübungspunkte auftreten als beim jeweiligen reinen Mineralöl.

EP 0 293 715 A2

Vergleichsbeispiel IX

Entsprechend der EP-OS 0 064 236 wurde ein niedriger und ein höher viskoser Polyether hergestellt und deren Viskosität und Verdampfungsverlust (Noack-Test gemäß DIN 51 581) bestimmt.

a) Ein mit Isotridecanol gestarteter Polyether hatte bei 40°C eine Viskosität von 96,7 mm²/s und einen Verdampfungsverlust von 17,5 Gew.-%.

b) Ein mit der entsprechenden molaren Menge Butandiol-1,4 (anstatt Isotridecanol) gestarteter und sonst identisch mit a) hergestellter Polyether hatte bei 40°C eine Viskosität von 240 mm²/s und einen Verdampfungsverlust von 9,5 Gew.-%.

Man erkennt, daß niedrigviskose Polyether entsprechend EP-OS 0 064 236 einen hohen Verdampfungsverlust aufweisen.

Vergleichsbeispiel X

An zwei Schmierölen gemäß dem Stand der Technik wurden anwendungstechnisch relevante Parameter bestimmt.

Vergleichsbeispiel Xa

455,3 g Propylenglykol wurden mit 66,7 g 45 %iger wäßriger Kalilauge vermischt und aus der Mischung das Wasser durch Erwärmen bis 110°.C bei einem Druck von 1 h Pa entfernt. Dann wurden innerhalb von 6 Stunden bei 105°C 5544,7 g Propylenoxid zugefügt, danach durch zugabe von 218,3 g 12 %iger Schwefelsäure und 600 g Wasser neutralisiert. Nach Abdestillieren des Wassers wurde noch 3 Stunden auf 105°C bei einem Druck von 1 h Pa erhitzt, unter Stickstoff abgekühlt und über ein Anschlemmfilter abgedrückt. Es wurden 5800 g eines Produktes mit einer OH-Zahl von 112 erhalten. Das produkt entspricht der DE-AS 1 130 176.

## Vergleichsbeispiel Xb

Handelsübliches Mineralöl Typ HL-Öl Verdichteröl 68.
Die Meßergebnisse sind aus der Tabelle 3 ersichtlich.

### Tabelle 3

| | Vergleichs- beispiel Xa | Vergleichs- beispiel Xb |
|---|---|---|
| Viskosität bei 40° C [mm²/s] | 68 | 70,9 |
| Viskosität bei 100° C [mm²/s] | 11 | 8,9 |
| Pour Point (°C) | -42 | -19 |
| Viskositätsindex VIE | 150 | 98,0 |
| Verdampfungsverlust nach DIN 51 581 (Gew.-%) | 21,7 | 5,0 |
| Almen-Wieland-Test (Werte an der Belastungsgrenze) | | |
| Schweißkraft (N) | 10000 | 1500 |
| Reibungskraft (N) | 2700 | 300 |
| Temperatur (°C) | 94 | 24 |
| Reichert-Reibverschleiß-Test (bei 15 N Belastung) | | |
| Verschleißmarke (mm²) | 11,6 | 26,3 |
| spez. Belastbarkeit (MPa) | 25,8 | 11,4 |
| Löslichkeit in Mineralölen (Zusatz 50 Vol.-%) | | |
| zu Paraffin-basischem Grundöltyp klar bis (°C) | > +20 | -17 |
| zu Gemisch-basischem Grundöltyp klar bis (°C) | > +20 | -14 |
| zu Naphthen-basischem Grundöltyp klar bis (°C) | > +20 | -27 |
| zu Poly-α-olefin Grundöltyp klar bis (°C) | > +20 | -16 |

### Erläuterungen zu Tabelle 3:

Es ist ersichtlich, daß das Polyetherschmieröl entsprechend DE-AS 1 130 176 (siehe Vergleichsbeispiel Xa) bei gegebener Viskosität eine Kombination von schlechtem Verdampfungsverlust, guten Almen-Wieland- und Reichert-Tests und nicht vorhandener Mineralölmischbarkeit darstellt, während das handelsübliche Mineralöl (siehe Vergleichsbeispiel Xb) bei gegebener Viskosität eine Kombination aus brauchbarem Verdampfungsverlust, schlechten Almen-Wieland- und Reichert-Tests und brauchbare Mineralölmischbarkeit darstellt. Demgegenüber zeichnen sich erfindungsgemäße Polyether (siehe Tabelle 1, Beispiele 1 und 5) durch gute Werte bei allen anwendungstechnisch relevanten Parametern aus, d.h. bei ihnen sind bei gegebener Viskosität ein niedriger Verdampfungsverlust mit guten Almen-Wieland- und Reichert-Tests sowie einer guten Mineralölmischbarkeit kombiniert.

## Ansprüche

1. Hydroxylgruppen-monofunktionelle Polyether, dadurch gekennzeichnet, daß sie als eingebaute Endgruppen oder Monomereinheiten

a) 1 bis 30 Gew.-% eines oder mehrerer $C_4$- bis $C_{24}$-Alkylmonophenole,

b) 1 bis 30 Gew.-% eines oder mehrerer $C_8$- bis $C_{24}$-Monoalkanole,

c) 1 bis 30 Gew.-% eines oder mehrerer $C_{10}$- bis $C_{20}$-1,2-Epoxyalkane und

d) 45 bis 80 Gew.-% Propylenoxid oder eine überwiegend aus Propylenoxid bestehende Niederalkylenoxidmischung

enthalten, wobei die Summe der Komponenten a) bis d) 100 Gew.-% ergibt,

und daß sie mittlere Molmassen von 600 bis 2500 aufweisen.

2. Polyether nach Anspruch 1, dadurch gekennzeichnet, daß der Anteil der Komponenten a) und b) jeweils 5 bis 15 Gew.-%, der Anteil der Komponente c) 5 bis 20 Gew.-%, der Anteil der Komponente d) 50 bis 65 Gew.-% und die mittlere Molmasse 700 bis 1500 betragen.

3. Polyether nach Ansprüchen 1 und 2, dadurch gekennzeichnet, daß bei Komponente a) der Alkylsubstituent oder, bei mehreren Alkylsubstituenten derjenige mit der längsten Kohlenstoffkette sich in Bezug auf die phenolische OH-Gruppe in m- oder p-Position befindet, daß es sich bei Komponente b) um n-Octanol, Isooctanol, Isononanol, Decanole, Dodecanole, Isotridecanol, Cetylalkohol, Stearylalkohol, $C_{12}$-bis $C_{20}$-Oxoalkohole oder $C_{16}$-bis $C_{24}$-Guerbetalkohole handelt, daß es sich bei der Komponente c) um 1,2-Epoxydecan, 1,2-Epoxydodecan, 1,2-Epoxyhexadecan, 1,2-Epoxyoctadecan oder Mischungen davon handelt und, daß es sich bei Komponente d) um Propylenoxid handelt.

4. Verfahren zur Herstellung von Polyethern des Anspruchs 1, dadurch gekennzeichnet, daß man die Komponente a) und die Komponente b) zusammen in dem Molverhältnis als Starter für eine anionische Epoxidpolymerisation mit den Epoxiden entsprechend der Komponenten c) und d) einsetzt, wie es auch als Molverhältnis der Monoetherendgruppen im Polyether-Endprodukt gewünscht wird.

5. Verfahren nach Anspruch 4, dadurch gekennzeichnet, daß man flüchtige Beimengungen und Verunreinigungen durch Strippen, Nachbehandeln in einen Dünnschichtverdampfer oder Ausdestillieren im Hochvakuum entfernt.

6. Schmiermittel, dadurch gekennzeichnet, daß sie Polyether des Anspruchs 1 enthalten.

7. Schmiermittel nach Anspruch 6, dadurch gekennzeichnet, daß sie zusätzlich Additive, andere Polyether als diejenigen des Anspruchs 1, Mineralöle, Poly-α-olefine und/oder Schmierstoffe auf der Basis von Estern, Phosphaten, Glykolen und/oder Polyglykolen enthalten.

8. Schmiermittel nach Ansprüchen 6 und 7, dadurch gekennzeichnet, daß sie 50 bis 95 Gew.-Teile einer Mineralölfraktion mit Schmierviskosität oder eines Poly-α-olefins, 5 bis 50 Gew.-Teile Polyether des Anspruchs 1 und 0 bis 10 Gew.-% übliche Schmierstoffadditive enthalten.

9. Schmiermittel nach Ansprüchen 6 bis 8, dadurch gekennzeichnet, daß sie zusätzlich Antioxidantien, Metallpassivatoren, Rostinhibitoren, Viskositätsindex-Verbesserer, Stockpunkterniedriger, Dispergiermittel, Detergentien, Hochdruckzusätze und/oder Verschleißadditive enthalten.

10. Schmiermittel nach Ansprüchen 6 bis 9, dadurch gekennzeichnet, daß sie insgesamt nicht mehr als 10 Gew.-% Additive und nicht mehr als 3 Gew.-% eines einzelnen Additivs enthalten.

FIG. 1

FIG. 2

FIG. 3

FIG. 4